Europäisches Patentamt

European Patent Office

Office européen des brevets

(11) Publication number: **0 632 265 A2**

## EUROPEAN PATENT APPLICATION

(21) Application number: 94109919.4

(22) Date of filing: 27.06.94

(51) Int. Cl.6: **G01N 27/00**

(30) Priority: **30.06.93 JP 160985/93**

(43) Date of publication of application:
**04.01.95 Bulletin 95/01**

(84) Designated Contracting States:
**DE FR GB IT**

(71) Applicant: **OSAKA GAS CO., LTD.**
**1-2, Hiranomachi 4-chome**
**Chuo-ku**
**Osaka-shi**
**Osaka 541 (JP)**
Applicant: **INTERNATIONAL**
**SUPERCONDUCTIVITY TECHNOLOGY**
**CENTER**
**No. 34-3, Shinbashi 5-chome**
**Minato-ku**
**Tokyo (JP)**
Applicant: **MITSUBISHI DENKI KABUSHIKI**
**KAISHA**
**2-3, Marunouchi 2-chome**
**Chiyoda-ku**
**Tokyo (JP)**

(72) Inventor: **Kudo, Shuzo**
**2-47-406, 3-chome,**
**Ohmiya-cho**
**Nara-shi,**
**Nara-ken (JP)**
Inventor: **Ohnishi, Hisao**
**3-17-4-19, Torishima**

**Konohama-ku,**
**Osaka (JP)**
Inventor: **Ipponmatsu, Masamichi**
**8-17-202, Kohien Sunada-cho**
**Nishinomiya-shi,**
**Hyogo-ken (JP)**
Inventor: **Takano, Satoshi**
**2-5-10-202, Sanuki**
**Ryugasaki-shi,**
**Ibaragi-ken (JP)**
Inventor: **Wakata, Mitsunobu**
**2-4-5-403, Oyama**
**Sagamihara-shi,**
**Kanagawa-ken (JP)**
Inventor: **Yamauchi, Hisao**
**2-409, Ka 750**
**Nagareyama-shi,**
**Chiba-ken (JP)**
Inventor: **Tanaka, Shoji**
**2-5-25-103, Kitashinjuku**
**Shinjuku-ku,**
**Tokyo (JP)**

(74) Representative: **Brommer, Hans Joachim,**
**Dr.-Ing. et al**
**Patentanwälte Dipl.-Ing. R. Lemcke,**
**Dr.-Ing. H.J. Brommer,**
**Dipl.-Ing. F. Petersen**
**Postfach 4026**
**Bismarckstrasse 16**
**D-76025 Karlsruhe (DE)**

(54) Nitrogen oxide detecting sensor and method of manufacturing the same.

(57) A nitrogen oxide detecting sensor includes a gas detecting portion and electrodes electrically connected to the gas detecting portion. The gas detecting portion is composed mainly of an oxide compound having electric conductivity or semiconductivity. The oxide compound has a crystal structure of 2212 phase or resistivity ranging not more than 50 $\Omega$ cm. And, the oxide compoud is expressed generally as:

$Bi_2 Sr_2 (Ca_{1-x} Y_x) Cu_2 O_{8 \pm \delta}$

(where, X is more than 0 but not more than 1 excluding 1, $\delta$ is more than 0 but less than 1 including 1).

Rank Xerox (UK) Business Services
(3.10/3.09/3.3.4)

EP 0 632 265 A2

FI G. 1

BACKGROUND OF THE INVENTION

1. FIELD OF THE INVENTION

The present invention relates to a nitrogen oxide detecting sensor and a method of manufacturing the sensor and more particularly to a nitrogen oxygen sensor to be used in the art of reducing or decomposing nitrogen oxide and a method of manufacturing such sensor.

2. DESCRIPTION OF THE RELATED ART

As the conventional methods of measuring a concentration of nitrogen oxide in exhaust gas, there are known the chemiluminescence method, infrared absorbing analysis method, ultraviolet ray absorbing analysis method, controlled potential electrolysis method and controlled potential coulomb method.

Further, in recent years, there is a growing scientific interest to employ, as an oxide semiconductor sensor, a compound expressed by;

$$YBa_2\ Cu_3\ O_7 - \delta$$

At present, the chemiluminescence method is considered as the most promising of all for its highest measurement precision and reliability. However, this method requires such components as an ozone generator, a photomultiplier tube, and a high voltage source. Thus, this method suffers the limitations in the possibility of system size reduction, cost reduction and maintenance conditions.

When a high detection precision is not needed, the controlled potential electrolysis method is convenient. This method, however, suffers the problems of secular change of the electrodes and maintenance of electrolysis solution.

Moreover, the devices employed by these methods cost hundreds or thousands of dollars. Also, while the measurement precisions are high, the methods require calibration due to occurrence of drift and the devices employed have the problem of durability.

On the other hand, the above-described oxide semiconductor sensor has the problem that its sensitivity characteristics are subject to change if water is present in the detection environment.

Accordingly, the primary object of the present invention is to provide a nitrogen oxide sensor which is simple in its construction and which is economical and durable and to provide also a method of manufacturing such sensor.

SUMMARY OF THE INVENTION

For accomplishing the above-noted object, a nitrogen oxide detecting sensor, according to the present invention, comprises:

a gas detecting portion composed mainly of an oxide compound having electric conductivity or semiconductivity, said oxide compound having a crystal structure of 2212 phase and expressed generally as:

$$Bi_2\ Sr_2\ (Ca_{1-x}\ Y_x)\ Cu_2\ O_8 \pm \delta$$

(where, X is more than 0 but not more than 1 excluding 1, $\delta$ is more than 0 but less than 1 including 1) and

electrodes electrically connected to said gas detecting portion.

According to one aspect of the present invention, a nitrogen oxide detecting sensor comprises:

a gas detecting portion comprised mainly of an oxide compound having electric conductivity or semiconductivity and resistivity ranging not more than 50 $\Omega$ cm and expressed generally as:

$$Bi_2\ Sr_2\ (Ca_{1-x}\ Y_x)\ Cu_2\ O_8 \pm \delta$$

(where, X is more than 0 but not more than 1 excluding 1, $\delta$ is more than 0 but less than 1 including 1) and

electrodes electrically connected to said gas detecting portion.

Functions and effects of these constructions will be described next.

The oxide compounds used in the gas detecting portion of these sensors include, in their crystal structures, a two-dimensional copper-oxygen chain structure, to or from which chain structure nitrogen oxide molecules are reversibly coupled or uncoupled. This reaction occurs according to the concentration of the nitrogen oxide in the gas phase, and the reaction comprises a reversible coupling/uncoupling reaction. With occurrence of this reaction, the gas detecting portion experiences a change in its ohmic resistance value according to the concentration of the nitrogen oxide in the gas phase. This resistance change is detected by the electrodes of the sensor, so that the sensor functions as a nitrogen oxide detecting sensor.

Then, even if the oxide compound satisfies the requirement of the above-described general composition formula, if this compound has a crystal structure other than 2212 phase or a resistivity more than 50 $\Omega$ cm (as will be described in sample experiments to follow, if the crystal structure includes any other phase than the 2212 phase, its resistivity too substantially satisfies this condition), the sensitivity tends to be lower. Accordingly, such sensor is not useful. Therefore, it order for the sensor to work effectively, this sensor needs to satisfy the above-described conditions.

As described above, with the sensor according to the present invention, through the measurement of a change in the ohmic resistance in the gas detecting portion via the electrodes, it is possible to detect, with practically sufficient sensitivity, the concentration of nitrogen oxide gas alone included within an atmosphere or combustion exhaust gas. Further, since this sensor comprises such simple combination of oxide compound and electrodes, the present invention has achieved a sensor which is constructionally simple and inexpensive and also a method of manufacturing such sensor.

Further and other objects, features and effects of the invention will become more apparent from the following more detailed description of the embodiments of the invention with reference to the accompanying drawings.

BRIEF DESCRIPTION OF THE DRAWINGS

Fig. 1 is a view showing a construction of a sensor according to one preferred embodiment of the present invention,
Figs. 2(a), (b), (c) and (d) show sensitivity characteristics of the sensor to target gas of various temperatures,
Fig. 3 is a view showing gas selectivity of the sensor,
Fig. 4 is a view illustrating relationship between sensitivity and resistivity of materials having different crystal structures,
Figs. 5(a) and (b) are views illustrating reversible characteristics of the sensor having materials of different crystal structures,
Fig. 6 is a view showing secular change in the sensitivity of the oxide compound,
Fig. 7 is a view showing a construction of a sensor according to a further embodiment of the invention, the sensor having an oxidation catalyst layer, and
Fig. 8 is a view showing a construction of a sensor according to a still further embodiment of the invention.

DESCRIPTION OF THE PREFERRED EMBODIMENTS

Preferred embodiments of a nitrogen oxide sensor relating to the present invention will be described next with reference to the accompanying drawings. The embodiments will be described in the order of constructions, manufacturing methods and characteristics of the sensors.

1. sensor constructions

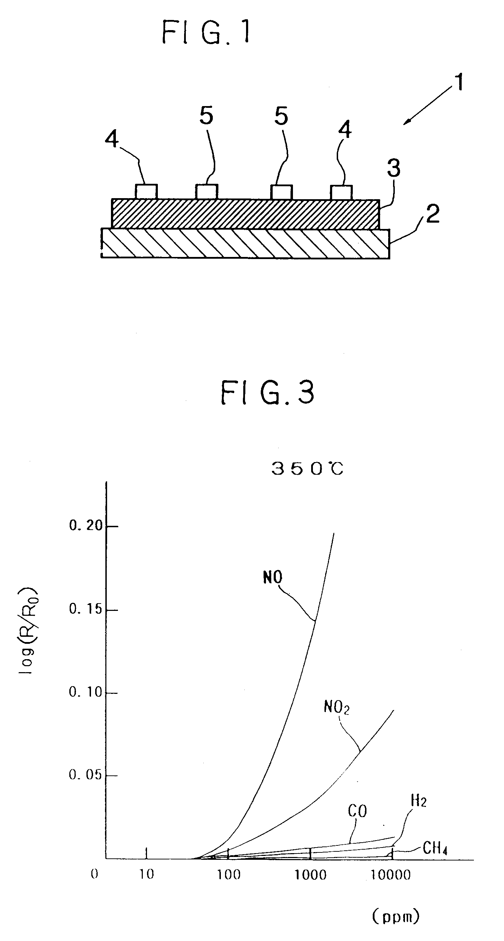

Fig. 1 shows a construction of a nitrogen oxide detecting sensor 1 according to one preferred embodiment. This nitrogen oxide detecting sensor 1 includes a heating base plate 2 comprised of a ceramic heater plate and a lump-shaped gas detecting portion 3 formed on the base plate 3 and comprised of an oxide compound of predetermined composition according to the present invention. For this gas detecting portion 3, there are provided a pair of current applying Pt electrodes 4 and a corresponding pair of potential detecting electrodes 5 for the current applying electrodes 4. In the following description, the gas detecting portion 3 is formed as a lump-shaped portion. Alternately, this gas detecting portion 3 may be formed as a thin film.

The gas detecting portion 3 is formed mainly of:

$$Bi_2\ Sr_2\ (Ca_{1-x}\ Y_x)\ Cu_2\ O_{8\ \pm\ \delta}$$

(where, X is more than 0 but not more than 1; $\delta$ is more than 0 and less than 1).

With this sensor construction, a variation in ohmic resistance of the gas detecting portion 3 is detected.

As to the electrode construction, with the above-described construction, electric current runs also through a variable resistance portion of the gas detecting portion, such that it is possible to effectively detect a change in the ohmic resistance associated with presence of nitrogen oxide gas.

2. manufacturing methods of the sensor

Next, methods of manufacturing the oxide compound constituting the gas detecting portion 3 will be described.

Powderly materials of $Bi_2\ O_3$, $SrCO_3$, $CaCO_3$, $Y_2\ O_3$ and either Cu or CuO are mixed in a predetermined mol ratio and this mixture is calcinated in the air at 800 degrees in Celsius for a period of 20 hours. Thereafter, this calcinated mixture is pulverized, shaped and sintered under the following conditions.

Table 1 shows the sintering conditions provided respectively for oxide compounds (to be referred to as Samples 1 through 5) obtained by substituting values '0', '0.2', '0.4', '0.6' and '0.8' for the variable 'x' in the above chemical formula.

The electrodes 4, 5 are provided to the gas detecting portion 3, and there is also provided the heating base plate 2 under the gas detecting portion 3.

Incidentally, the compounds are not limited to the above-identified kinds, i.e. $Bi_2\ O_3$, $Sr\ CO_3$, $CaCO_3$, $Y_2\ O_3$ and CuO. Various other kinds of compounds obtainable from a variety of combinations of the mentioned elements may be employed as well. Such other kinds of compounds include, for instance, various kinds of inorganic salts, organic salts, oxides, chlorides and hydroxides.

3. characteristics of the sensors

The sensitivity characteristics of the sensors manufactured in the above-described manner were evaluated as follows.

A fixed electric potential was applied to the heating base plate 2. While the gas detecting portion was maintained at 250, 300, 350 and 400 degrees in Celsius, respectively. an electric current of 10 mA was applied to the current-applying electrodes 4. Air containing a variety of components at predetermined concentrations was caused to contact the gas detecting portion, and electric potentials were measured through the potential detecting electrodes 5, thereby to obtain ohmic resistance values.

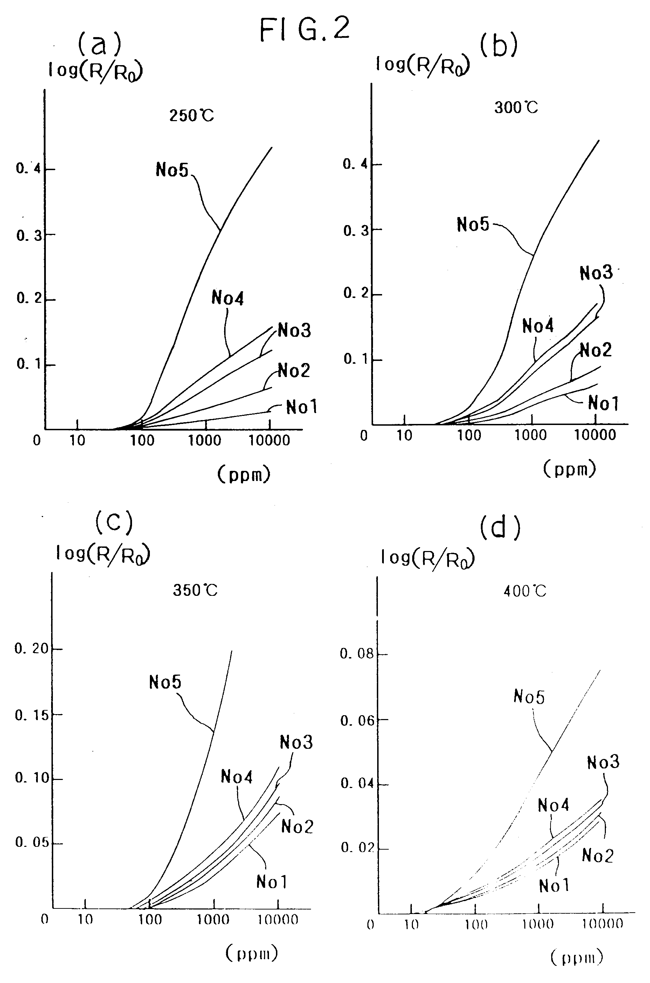

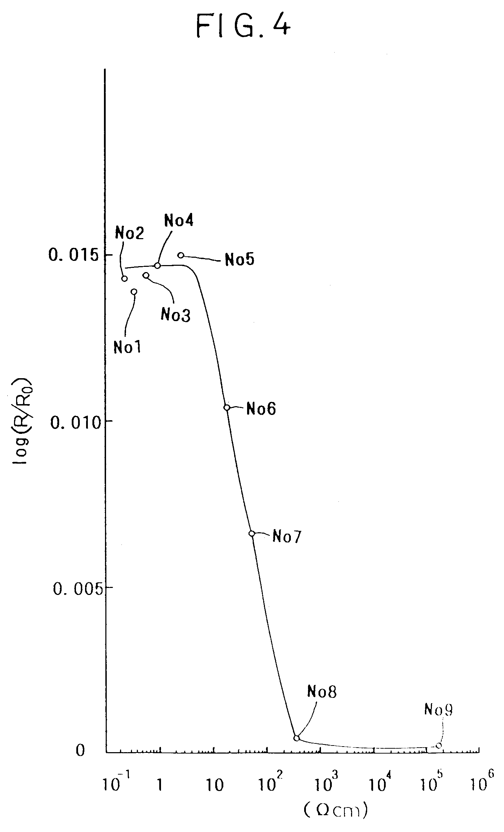

Fig. 2 shows the NO sensitivity characteristics of the Samples No. 1 through 5 identified in Table 1 at the respective temperatures (i.e. 250, 300, 350 and 400 degrees in Celsius). Fig. 3 shows the sensitivity characteristics of the Sample No. 5 at the sensor temperature of 350 degrees in Celsius with respect to various gases (NO, $NO_2$, CO, $H_2$, $CH_4$). In these graphs, the resistance values in the air are denoted by $R_\theta\ (\Omega)$ and those in the other gases are denoted by $R\ (\Omega)$. Further, the horizontal axes represent the concentrations of the gases (unit = ppm), while the vertical axes represent the sensitivities ($\log\ (R/R_0)$).

As described hereinbefore, for each of the Samples Nos. 1 through 5, the ratio of yttrium contained in the respective oxide compounds was increased from '0' to '0.8', in correspondence with the order of the numbering of the samples.

As the results, it was found that these sensors provided sufficient sensitivities to NO and $NO_2$. As may be seen from Figs. 2(a), (b), (c) and (d), in terms of the content ratio of yttrium (Y), the Sample No. 5 was found as best. And, this sensor was found to achieve the highest performance at the temperature of 350 degrees in Celsius. Further, as shown in Fig. 3, these sensors provided satisfactory gas selectivities to the other interfering gases.

## TABLE 1

| sample No. | X | composition | sintering conditions |
|---|---|---|---|
| 1 | 0 | $Bi_2Sr_2CaCu_2O_{8+\delta}$ | 840°C 30hr in air |
| 2 | 0.2 | $Bi_2Sr_2(Ca_{0.8}Y_{0.2})Cu_2O_{8+\delta}$ | 870°C 30hr in air |
| 3 | 0.4 | $Bi_2Sr_2(Ca_{0.6}Y_{0.4})Cu_2O_{8+\delta}$ | 910°C 30hr in air |
| 4 | 0.6 | $Bi_2Sr_2(Ca_{0.4}Y_{0.6})Cu_2O_{8+\delta}$ | 930°C 30hr in air |
| 5 | 0.8 | $Bi_2Sr_2(Ca_{0.2}Y_{0.8})Cu_2O_{8+\delta}$ | 930°C 30hr in air |

4. experiment examples

The present inventors conducted experiments on a variety of materials in order to find out suitable materials as the oxygen compounds forming the gas detecting portion 3. These experiments will be described next.

Table 2 shows composition formulas and crystal structures of nine types of oxide compounds evaluated. In this table, the Samples Nos. 1 through 5 are identical to those shown in Table 1.

These compounds have crystal structures comprised solely of 2212 phase of BSCCO (see "Japan Journal of Applied Physics" M. Onoda, A. Yamamoto, E. Takayama-Muromachi and S. Takekawa vol. 27 1988 L833).

On the other hands, further Samples Nos. 6 through 9 contain, in addition to the above-identified phase, other oxide compounds comprised of bismuth (Bi), strontium (Si), and copper (Cu). Here, if x = 1 (i.e. no Ca is contained) in the foregoing formula, the crystal structure does not comprise the 2212 phase. The structures were determined by means of X-ray diffraction analysis.

TABLE 2

| sample No. | composition | structure |
|---|---|---|
| 1 | $Bi_2 Sr_2 Ca Cu_2 O_{8\pm\delta}$ | BSCCO 2212 phase alone |
| 2 | $Bi_2 Sr_2 (Ca_{0.8}Y_{0.2}) Cu_2 O_{8\pm\delta}$ | BSCCO 2212 phase alone |
| 3 | $Bi_2 Sr_2 (Ca_{0.6}Y_{0.4}) Cu_2 O_{8\pm\delta}$ | BSCCO 2212 phase alone |
| 4 | $Bi_2 Sr_2 (Ca_{0.4}Y_{0.6}) Cu_2 O_{8\pm\delta}$ | BSCCO 2212 phase alone |
| 5 | $Bi_2 Sr_2 (Ca_{0.2}Y_{0.8}) Cu_2 O_{8\pm\delta}$ | BSCCO 2212 phase alone |
| 6 | $Bi_2 Sr_2 Ca Cu_2 O_{8\pm\delta}$ | 2212 phase and trace amount of oxide compound of Bi, Sr, Cu |
| 7 | $Bi_2 Sr_2 Ca Cu_2 O_{8\pm\delta}$ | 2212 phase and trace amount of oxide compound of Bi, Sr, Cu |
| 8 | $Bi_2 Sr_2 Ca Cu_2 O_{8\pm\delta}$ | trace amount of 2212 phase and oxide compound of Bi, Sr, Cu and oxide compound of Ca, Cu |
| 9 | $Bi_2 Sr_2 Ca Cu_2 O_{8\pm\delta}$ | trace amount of 2212 phase and oxide compound of Bi, Sr, Cu and oxide compound of Ca, Cu |

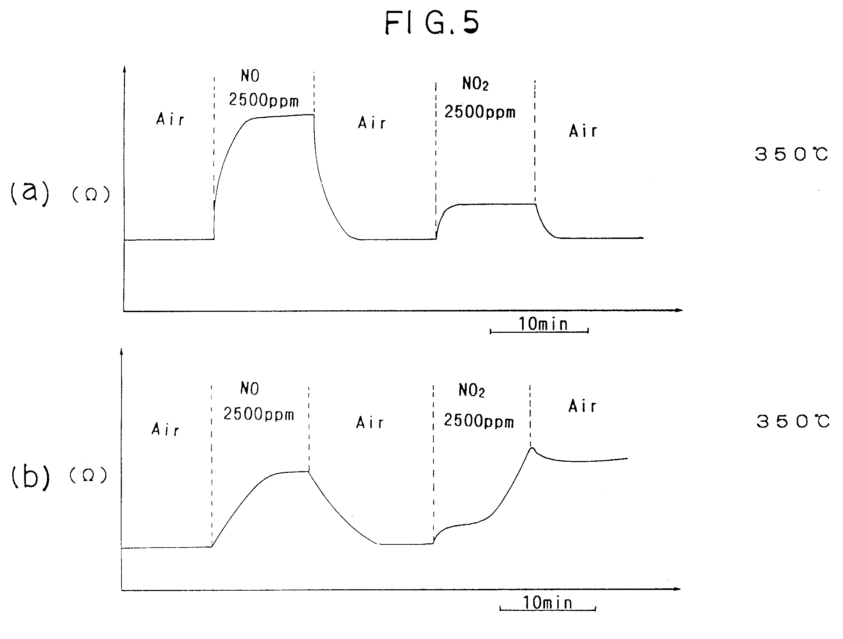

Fig. 4 show relationships between the resistivities and the sensitivities of the above-described Samples Nos. 1 through 9. Further, Fig. 5 show sensor reversibility characteristics of the Sample sensors Nos. 5 and 8 with respect to NO gas and NO gas.

In Figs. 5, Fig. 5 (a) shows the results of the Sample No. 5 and Fig. 5(b) shows the results of the Sample No. 8. In these drawings, the horizontal axes represent time lapse while the vertical axes represent change in the ohmic resistance values ($\Omega$). In the earlier stages of the experiments, the sensors were caused to contact NO gas. Then, after contact with air, the sensors were caused to contact $NO_2$ gas.

From Fig. 4, it may be understood that the sensitivities to the nitrogen oxides vary depending on the sample structures. As shown in Fig. 4, higher sensitivity was obtained from the Samples Nos. 1 through 5 having crystal structures comprised of 2212 phase. Concerning the resistivities, those having resistivities less than 50 $\Omega$ cm achieved satisfactory sensitivities.

Referring now to Fig. 5, the Sample No. 5 showed reversible sensitivity to both NO gas and $NO_2$ gas. Whereas, the Sample No. 8 did not show reversible sensitivity to $NO_2$ gas.

To consider the possible reason for the above fact, the Sample No. 5 is comprised solely of 2212 phase, while the Sample No. 8 includes the other phases than 2212 phase such as the oxide compound of Ca and Cu. Then, in this Sample No. 8, the oxide compound of Ca and Cu irreversibly absorbs $NO_2$ , thereby to prevent the ohmic resistance of this Sample No. 8 from restoring to its initial value. The present inventors conducted further similar experiments on the Samples Nos. 1 through 4, 6, 7 . In these experiments too, the above-described relationship between the crystal structure and the ohmic resistance value applied.

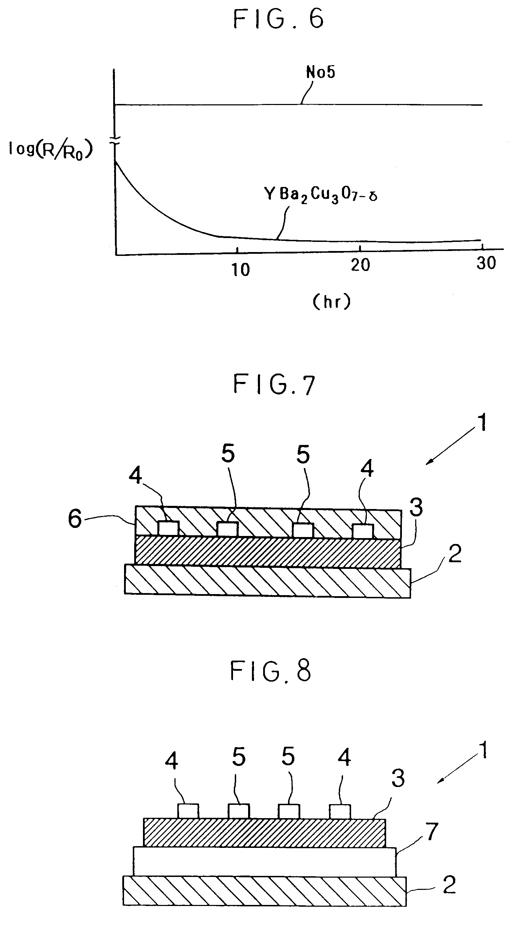

As to the durability of the sensor, as shown in Fig. 6, the conventional type expressed by the formula:

$$YBa_2\ Cu_3\ O_{7\ -\ \delta}$$

loses its sensitivity substantially entirely after a period of 10 hours under a humidified condition. In contrast, in the case of the sensors according to the composition of the present invention, there occurs no secular change from initial value thereof. Accordingly, it may be considered that the sensor according to the present invention provides sufficient durability.

Next, some other experiment examples will be described.

(1) construction having an oxidation catalyst layer 6:

In this case, the sensor 1 having the aforedescribed construction is provided further with an oxidation catalyst layer 6 including an element belonging in the platinum group, such as platinum (Pt), palladium (Pd) or the like, for eliminating, through an oxidation reaction, the above-identified interfering components (CO, $H_2$ , $CH_4$ and also $C_2\ H_5\ OH$, $CH_3\ OH$ or the like). This sensor too is effective for detection of nitrogen oxides. The construction of this sensor is shown in Fig. 7.

As one method to form this oxidation catalyst layer 6, substance comprised of activated alumina dispersedly mixed with about 1 % of the platinum group element is screen-printed, in the thickness of 500 $\mu$m approximately, on the film of the gas detecting portion 3 and then sintered.

In addition to the above-described construction, further constructions may be obtained by:

(a) adding and mixing about 0 to 2 % of a platinum group element such as Pt, Pd or the like to the oxide compound; or

(b) causing a platinum group element such as Pt, Pd or the like to be carried on the surface of the oxide compound.

(2) electrode constructions

In the foregoing embodiment, the pair of current-applying electrodes 4 and the the pair of potential-detecting electrodes 5 are provided for detecting change in the ohmic resistance value. Instead, it is conceivable to apply a fixed potential between a pair of potential-applying electrodes and to detect an electric current between a pair of current-detecting electrodes.

As a further electrode construction, it is possible to employ conventional two-terminal electrodes (each acting as both a current electrode and a potential electrode). In this case, however, at a part of the gas detecting portion, there occurs a partial resistance change due to absorption of nitrogen oxide, so that the electric current predominantly flows only through a lower resistance part. Accordingly, the change in the resistance value at the gas detecting portion detectable through the electrodes may be very small. In this sense, the constructions described in the above embodiments are more desirable.

However, any other type of electrode construction will be possible as long as the construction allows effective detection of change in the resistance value taking place at the gas detecting portion.

(3) heating means

In order to improve the responsiveness and restorability of the ohmic resistance value of the nitrogen oxide sensor in response to a change in the nitrogen oxide gas concentration, it is preferred that the sensor be heated higher than 250 degrees in Celsius as described in the foregoing embodiment. For this purpose, any appropriate heating means such as the ceramic heating plate employed in the described embodiment should be provided. As this heating means, it is also conceivable to surround the entire sensor with a heater coil or to surround only a portion of the sensor with such heater coil with the remaining portion thereof being heated by the ceramic heater plate.

The provision of the heating means may sufficiently accelerate the absorption and discharge of the nitrogen oxide, thereby to improve the responsiveness and restorability of the ohmic resistance value of the nitrogen oxide sensor in response to a change in the nitrogen oxide gas concentration.

However, such heating means will not be needed for the detection as long as a sufficient amount of change in the resistance value may be obtained. Further, a self-heating type construction is conceivable in which the base plate per se is supplied with electric current to be heated thereby.

(4) sensor configuration

The configuration of the gas detecting portion 3 is not limited to the lump-like configuration described hereinbefore. Any other type of configuration is also possible such as a thin-film like configuration. With such configuration too, the gas detecting portion may provide the detecting function.

(5) methods of forming a thin film

As the method of forming a thin film of the gas detecting portion, in addition to the laser abrasion method, other methods are also possible such as the physical vapor deposition methods including the vacuum vapor deposition method and the sputtering method as well as the chemical vapor deposition methods including MO-CVD (Metal Organic Chemical Vapor Deposition) method and the chloride CVD method.

(6) As a still further embodiment of the present invention, the sensor may be constructed as shown in Fig. 8.

In the case of a nitrogen oxide detecting sensor 1 according to this embodiment, a gas detecting portion 3 in the form of a thin film is formed on one surface of a base plate 7. On the opposite surface of the base plate 7, there is disposed a heating base plate 2 incorporating a heater. Then, for this gas detecting portion 3, there are provided a pair of current-applying electrodes 4 and a corresponding pair of potential-detecting electrodes 5, in the same manner as the foregoing embodiment. In this embodiment too, the gas detecting portion 3 may be formed like a thin film. With this sensor construction also, nitrogen oxides may be effectively detected.

(7) In the case of the base plate 7 shown in Fig. 8, a poly-crystalline ceramic plate is generally used from the economic consideration. However, in case a higher sensor performance (higher sensitivity, a higher stability and so on) is desired. a single-crystalline ceramic plate may be used for forming the gas detecting portion 3 in the form of a thin film comprised of the oxide compound predetermined according to the present invention. As such single-crystalline ceramic plate, single-crystalline alumina (sapphire, $Al_2O_3$), single-crystalline magnesia (MgO), single-crystalline strontium titanate ($SrTiO_3$) or the like may be employed. In such case, because of absence of crystal grain phase, the thin-film gas detecting portion formed on the surface of the base plate will achieve a higher formation precision (especially, the precision in its thickness) and the thus-formed thin film will obtain more complete and defectless crystal structure. Consequently, a sensor of a higher performance may be obtained.

The invention may be embodied in other specific forms without departing from the spirit or essential characteristics thereof. The present embodiments are therefore to be considered in all respects as illustrative and not restrictive, the scope of the invention being indicated by the appended claims rather than the foregoing description and all changes which become within the meaning and range of equivalency of the claims are therefore intended to be embraced therein.

**Claims**

1. A nitrogen oxide detecting sensor having:

a gas detecting portion (3) composed mainly of an oxide compound having electric conductivity or semiconductivity; and

electrodes (4), (5) electrically connected to said gas detecting portion (3);

characterized in that

said oxide compound is expressed generally as:

$$Bi_2\ Sr_2\ (Ca_{1-x}\ Y_x)\ Cu_2\ O_{8\ \pm\ \delta}$$

(where, X is more than 0 but not more than 1 excluding 1, $\delta$ is more than 0 but less than 1 including 1); and in that

said oxide compound has either a crystal structure of 2212 phase or resistivity ranging not more than 50 $\Omega$ cm.

2. A nitrogen oxide detecting sensor as defined in Claim 1,
characterized in that
an oxidation catalyst layer (6) carrying a platinum group element including but not limited to Pt, Pd is provided at least on an outer side of said gas detecting portion (3).

3. A nitrogen oxide detecting sensor as defined in Claim 1,
characterized in that
a platinum group element including but not limited to Pt, Pd is added, by an amount of 0 to 2 %, to said oxide compound forming said gas detecting portion (3).

4. A nitrogen oxide detecting sensor as defined in Claim 1,
characterized in that
a platinum group element including but not limited to Pt, Pd is carried at least on an outer side of said gas detecting portion (3).

5. A nitrogen oxide detecting sensor as defined in Claim 1,
characterized in that
said gas detecting portion (3) comprises a thin film to be disposed on a heating base plate (2).

6. A nitrogen oxide detecting sensor as defined in Claim 5,
characterized in that
said heating base plate (2) comprises a ceramic plate.

7. A nitrogen oxide detecting sensor as defined in Claim 1,
characterized in that
said electrodes (4), (5) include a pair of current-applying electrodes (4) and a corresponding pair of potential-detecting electrodes (5).

8. A method of manufacturing a nitrogen oxide detecting sensor having the steps of:
fabricating a gas detecting portion having electric conductivity or semiconductivity; and
electrically connecting electrodes to said gas detecting portion;
characterized in that
said step of fabricating the gas detecting portion includes the steps of;
mixing, in a predetermined mol ratio, powderly materials of Bi compound, Sr compound, Ca compound, Y compound and either Cu or Cu compound, and
fabricating said gas detecting portion by sintering or heat-decomposing said mixture, said gas detecting portion being comprised mainly of an oxide compound having either a crystal structure of 2212 phase or resistivity ranging not more than 50 $\Omega$ cm, said oxide compound being expressed generally as:

$$Bi_2\ Sr_2\ (Ca_{1-X}\ Y_X)\ Cu_2\ O_{8\ \pm\ \delta}$$

(where, X is more than 0 but not more than 1 excluding 1, $\delta$ is more than 0 but less than 1 including 1).

9. A method as defined in Claim 15,
characterized by the further step of
attaching a base plate to a lower portion of said gas detecting portion.

10. A method as defined in Claim 9,
characterized in that
said base plate comprises a heating base plate.

10

**11.** A method as defined in Claim 9,
characterized by the further step of
attaching a heating base plate to a lower portion of said base plate.

**12.** A method of manufacturing a nitrogen oxide detecting sensor having the steps of:
fabricating a gas detecting portion having electric conductivity or semiconductivity; and
electrically connecting electrodes to said gas detecting portion;
characterized in that
said step of fabricating the gas detecting portion includes the step of;
forming said gas detecting portion on a base plate by means of any one of physical vapor deposition methods including the laser abrasion method, the vacuum vapor deposition method and the sputtering method or of chemical vapor deposition methods including the MO-CVD method and the chloride CVD method, said gas detecting portion being comprised mainly of an oxide compound having either a crystal structure of 2212 phase or resistivity ranging not more than 50 $\Omega$ cm, said oxide compound being expressed generally as:

$$Bi_2 Sr_2 (Ca_{1-X} Y_X) Cu_2 O_8 \pm \delta$$

(where, X is more than 0 but not more than 1 excluding 1, $\delta$ is more than 0 but less than 1 including 1).

**13.** A method as defined in Claim 12,
characterized in that
said base plate comprises a heating base plate.

**14.** A method as defined in Claim 12,
characterized by the further step of
attaching a heating base plate to a lower portion of said base plate.

# FIG.1

# FIG.3

# FIG. 2

(a)
log(R/R₀)

250℃

No5
No4
No3
No2
No1

0.4
0.3
0.2
0.1
0

0  10  100  1000  10000
(ppm)

(b)
log(R/R₀)

300℃

No5
No4
No3
No2
No1

0.4
0.3
0.2
0.1
0

0  10  100  1000  10000
(ppm)

(c)
log(R/R₀)

350℃

No5
No4
No3
No2
No1

0.20
0.15
0.10
0.05
0

0  10  100  1000  10000
(ppm)

(d)
log(R/R₀)

400℃

No5
No4
No3
No2
No1

0.08
0.06
0.04
0.02

0  10  100  1000  10000
(ppm)

13

# FIG. 4

# FIG.5

(a) (Ω)

Air | NO 2500ppm | Air | NO₂ 2500ppm | Air

350℃

|← 10min →|

(b) (Ω)

Air | NO 2500ppm | Air | NO₂ 2500ppm | Air

350℃

|← 10min →|

EP 0 632 265 A2

## FIG. 6

## FIG.7

## FIG.8